# EUROPEAN PATENT SPECIFICATION

(11) **EP 4 387 945 B1**
(45) Date of publication and mention of the grant of the patent: **24.06.2026**
(21) Application number: 22754234.7
(22) Date of filing: 19.07.2022
(51) Int. Cl.: C07C 6/04, C07C 5/333, C07C 11/107, C07C 11/06, C07C 11/08, C07C 11/10

(54) **METATHESIS OF C4/C5 TO PROPYLENE AND 1-HEXENE**
METATHESE VON C4/C5 ZU PROPYLEN UND 1-HEXEN
MÉTATHÈSE DE C4/C5 EN PROPYLÈNE ET 1-HEXÈNE

(30) Priority: 20.08.2021 US 202163235509 P
(43) Date of publication of application: 26.06.2024
(73) Proprietor: ExxonMobil Chemical Patents Inc., Baytown, TX 77520 (US)
(72) Inventor: DE SMIT, Emiel, 1200 Brussels (BE); LARSON, Robert, B., Houston, TX 77098 (US)
(74) Representative: ExxonMobil Petroleum & Chemical BV
(86) International application number: PCT/US2022/037574
(87) International publication number: WO 2023/022828

(56) References cited:
- FR-A1- 3 015 477
- US-A1- 2001 003 140
- US-B2- 6 580 009

## Description

### CROSS REFERENCE TO RELATED APPLICATIONS

This application claims the benefit of and priority to US Provisional Application No. 63/235,509 filed August 20, 2021.

### TECHNICAL FIELD

Exemplary embodiments described herein relate to a process and system for the simultaneous production of propylene and 1-hexene from a mixed C₄/C₅ alkane stream.

### BACKGROUND

European patent document EP2895445 describes a process for producing propylene, including: fractionating a mixed C₄ hydrocarbon stream to recover a first fraction comprising isobutene and a second fraction comprising 2-butene; contacting the first fraction with a first metathesis catalyst in a first metathesis reaction zone; recovering an effluent from the first metathesis reaction zone comprising at least one of ethylene, propylene, unreacted isobutene, C₅ olefins, and C₆ olefins; contacting the second fraction and the ethylene in the effluent with a second metathesis catalyst in a second metathesis reaction zone; recovering an effluent from the second reaction zone comprising at least one of unreacted ethylene, propylene, unreacted 2-butene, fractionating the effluent from the first metathesis reaction zone and the effluent from the second metathesis reaction zone to recover an ethylene fraction, a propylene fraction, one or more C₄ fractions, and a fraction comprising at least one of C₅ and C₆ olefins.

European patent document EP1831135 describes a process for producing propylene from a C₄ feed containing 2-butene, which includes contacting said feed with ethylene in a metathesis reaction zone containing a metathesis catalyst under metathesis reaction conditions to provide an effluent including propylene, said metathesis catalyst consisting essentially of a transition metal or oxide thereof supported on a high purity silica support possessing less than about 150 ppm magnesium, less than about 900 ppm calcium, less than about 900 ppm sodium, less than about 200 ppm aluminum, and less than about 40 ppm iron.

European patent document EP2552871 describes a process for the conversion, under conditions and with a catalyst system effective for olefin metathesis, of hydrocarbon feedstocks comprising butylene, for example all or a large proportion of a single C₄ olefin isomer such as butene-1, are described. Olefin products, and particularly propylene, are formed in the presence of a catalyst comprising a solid support and a tungsten hydride bonded to alumina present in the support. This occurs despite the expectation that the olefin metathesis reaction mechanism leads to the formation of olefin products having other carbon numbers.

French patent document FR3015477 describes a process for producing propylene from a feedstock comprising linear paraffins having a carbon number of from 8 to 30, said process comprising: a) a step of selective dehydrogenation of the linear paraffins of the feed, b) a step of isomerization and metathesis, in the presence of ethylene, linear monoolefins contained in the effluent from step a), c) a step of separating the effluent from step b). FR3015477 only focuses on propylene production by metathesis using dehydrogenated linear C₈-C₃₀ parrafins as a feed).

European patent document EP1134271 describes a process for preparing propene and hexene from a raffinate II feed stream comprising olefinic C₄-hydrocarbons comprises a) a metathesis reaction in which butenes present in the feed stream are reacted with ethene in the presence of a metathesis catalyst comprising at least one compound of a metal of transition groups VIb, VIIb or VIII of the Periodic Table of the Elements to give a mixture comprising ethene, propene, butenes, 2-pentene, 3-hexene and butanes, using, based on the butenes, from 0.05 to 0.6 molar equivalents of ethene, b) firstly fractionally distilling the product stream obtained in this way to give a low-boiling fraction A comprising C₂-C₃-olefins and a high-boiling fraction comprising C₄-C₆-olefins and butanes, c) subsequently fractionally distilling the low-boiling fraction A obtained from b) to give an ethene-containing fraction and a propene-containing fraction, with the ethene-containing fraction being returned to the process step a) and the propene-containing fraction being discharged as product, d) subsequently fractionally distilling the high-boiling fraction obtained from b) to give a low-boiling fraction B comprising butenes and butanes, a pentene-containing intermediate-boiling fraction C and a hexene-containing high-boiling fraction D, e) where the fractions B and C are completely or partly returned to the process step a) and the fraction D is discharged as product.

EP1483224 describes that a C₃ to C₆ hydrocarbon cut from a cracking unit is processed for the conversion of olefins to propylene and hexene via autometathesis. The autometathesis of a mixed normal butenes feed in the presence of a metathesis catalyst operates without any ethylene in the feed mix to the metathesis reactor. Some fraction of the 2-butene feed may be isomerized to 1-butene and the 1-butene formed plus the 1-butene in the feed react rapidly with the 2-butene to form propylene and 2-pentene. The feed to the reactor also includes the recycle of the 2-pentene formed in the reactor with unreacted butenes to simultaneously form additional propylene and hexene. In one embodiment, some or all of the 3-hexene formed in the reaction is isomerized to 1-hexene. In another embodiment, some portion of the 3-hexene produced in the main metathesis reaction is reacted with ethylene to produce 1-butene without the need for superfractionation. In another embodiment, the 3-hexene product is hydrogenated and recycled back to the cracking heaters.

### SUMMARY

A method, comprising: at least partially dehydrogenating a mixed alkane stream, including at least C₄ and C₅ alkanes, to generate at least a partial alkene stream and hydrogen; applying a metathesis reaction to the at least partial alkene stream to generate an effluent, wherein the metathesis reaction is optionally conducted in a presence of ethylene; and separating the effluent from the metathesis reaction into propylene and 1-hexene.

A system, comprising: a dehydrogenation unit configured to at least partially dehydrogenate a mixed alkane stream, including at least C₄ and C₅ alkanes, to generate at least a partial alkene stream and hydrogen; a metathesis unit configured to apply a metathesis reaction to the alkene stream to generate an effluent, wherein the metathesis reaction is optionally conducted in a presence of ethylene; and a separation unit configured to separate the effluent from the metathesis reaction into propylene and 1-hexene.

### BRIEF DESCRIPTION OF THE FIGURES

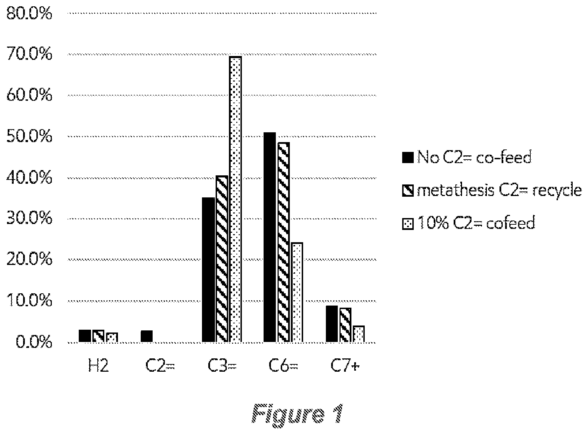

Figure 1 is a graph illustrating how co-feeding ethylene can shift product distrubtion.
Figure 2 illustrates an exemplary embodiment using a combination of dehydrogenation and metathesis to produce C₃⁼ and 1-C₆⁼.

### DETAILED DESCRIPTION

### Definitions

For the purposes of this invention and the claims thereto, the new numbering scheme for the Periodic Table Groups is used as described in Chemical and Engineering News, v.63(5), pg. 27 (1985). Therefore, a "group 4 metal" is an element from group 4 of the Periodic Table, e.g. Hf, Ti, or Zr.

As used herein, and unless otherwise specified, the term "Cₙ" means hydrocarbon(s) having n carbon atom(s) per molecule, wherein n is a positive integer.

The term "hydrocarbon" means a class of compounds containing hydrogen bound to carbon, and encompasses (i) saturated hydrocarbon compounds, (ii) unsaturated hydrocarbon compounds, and (iii) mixtures of hydrocarbon compounds (saturated and/or unsaturated), including mixtures of hydrocarbon compounds having different values of n. Likewise, a "Cm-Cy" group or compound refers to a group or compound comprising carbon atoms at a total number thereof in the range from m to y. Thus, a C₁-C₅₀ alkyl group refers to an alkyl group comprising carbon atoms at a total number thereof in the range from 1 to 50.

### Description

Natural gas liquids (NGL) are hydrocarbons such as propane, ethane, butane, pentanes, and other condenstates (C₅₊). They are hydrcocarbons removed (condensed) as a liquid from a hydrocarbon stream that is typically in a vapor phase (i.e., natural gas).

Propylene and 1-Hexene are important feedstocks for the chemical industry, with the majority being consumed for large scale polyolefin applcations such as polypropylene and linear low density ethylene-1-hexene copolymers (polyethylene, LLDPE).

Conventional steam cracking is generally used to produce lighter alkenes, such as ethylene and propylene. Steam cracking produces more kilograms (pounds) of ethylene to kilograms (pounds) of propylene on a weight basis.

A typical full range linear alpha olefin (LAO) plant yields Schulz-Flory type ethylene oligomer distributions, and as a result, typically has a relatively low specific yield of 1-hexene, producing many other LAOs, such as 1-butene, 1-octenes and C₁₀₊ LAOs in the process as major byproducts.

The present technological advancement advantageouosly can balance the above-noted mismatch of a steam cracker (low propylene) and an LAO plant (low 1-hexene) yield slate, and increase the yield of propylene and 1-hexene to meet growing market demand.

An embodiment of the present technological advancement can integrate the conversion of a mixed C₄ and C₅ alkane stream by dehydrogenation and subsequent metathesis into propylene and 1-hexene, where the final products are obtained by distillation (and isomerization to reach a higher yield of 1-hexene). Uncoverted olefins can be recycled back to the metatheis reactor. Advantageously, embodiments of the present technological advancement can include some or all of the following features: (a) using a mixed C₄/C₅ feed to make C₃⁼ and 1-C₆⁼ simultaneously (whereas a conventional approaches (EP1134271 and EP1483224) only used C₄ olefins in the metathesis); (b) at least partially dehydrogenating the mixed C₄/C₅ (NGL) feed to alkenes before converting to alkanes in metathesis (FR3015477 ony focuses on propylene production by metathesis using dehydrogenated linear C₈-C₃₀ parrafins as feed); (c) using any other mixed C₄/C₅ alkane/alkene streams (e.g., FCC (fluid catalytic cracking, LLCN (light-light cracked naphtha), or other sources) for the combined production of propylene and 1-hexene; (d) passing a mixed C₄/C₅ (linear and brached) alkane/paraffin mixture through a metathesis reactor (without cofeeding C₂⁼) to make two products (C₃= and 1-C₆=) simultaneously; (e) passing a mixed C₄/C₅ (linear and brached) alkane/paraffin mixture through a metathesis reactor while cofeeding C₂⁼ to enhance C₃⁼ yield vs. 1-C₆⁼ yield as desired (conventional processes noted in the Background section describes using linear and highly olefinic feedstocks and not using mixed branched/linear and paraffinic/olefinic feedstock, wherein branched isomers and paraffins pass through the metathesis process and can be separated by distillation after the metathesis reactor and recycled back to the dehydrogenation reactor).

Olefin metathesis can be an attractive technology to convert heavier, less desirable olefins into ethylene and propylene, for which there is a larger market demand. For example, C₄ olefins can be converted to propylene using ethylene.

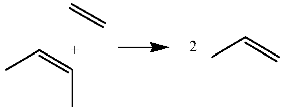

Conceptually, the present technological advancement can convert raffinate-2 from e.g. FCC units (or steam crackers) to propylene and therefore improve the overall C₃= : C₂= balance of olefin plants. In the same manner, mixed butenes (i.e. 1-butene and 2-butene) can also be converted to a mixture of ethylene, propylene, 2-pentene and 3-hexene.

The same metathesis chemistry also allows to make on-purpose 1-hexene (LLDPE co-monomer) from butenes:

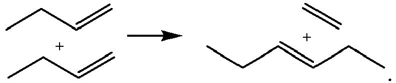

In this reaction, 3-hexene and ethylene are formed in the metathesis reaction from 1-butenes, and the 3-hexene is then isomerized into 1-hexene, 2-hexene and 3-hexene from which 1-hexene can be purified by distillation where the undesired other hexenes recycled to extinction.

Thus, conceptually, metathesis allows the conversion of terminal and internal olefins of a narrow (e.g. less desired, heavy) carbon number range into a mixture of terminal and internal olefins of a broader (including smaller, more desired) carbon number range.

The present technological advancement can integrate the conversion of C₄ and C₅ (e.g. NGL) feed into a mixed C₄ / C₅ olefin stream, which is then converted by metathesis into mainly propylene and 1-hexene by distillation and recycling. The present technological advancement provides a way rectifying the propylene (in the case of steam cracker yield slate) and 1-hexene (in the case of full range LAO plant yield slate) production and market demand by the on-purpose production of propylene and 1-hexene from C₄ / C₅ feeds.

Although exemplary embodiments of the present technological advancement focuses on the use of C₄ / C₅ NGL feed, converted by dehydrogenation to provide the olefinic feed needed for the metathesis reaction, it is noted here that this process can also be applied to any existing olefinic C₄₊ streams (FCC light cracked naphtha or raffinate, Steam cracker raffinate, butenes and pentenes from biomass sources, etc.).

Several olefin metathesis technologies have been commercialized (e.g. Shell Higher Olefin Process, Axens Meta-4, Lummus OCT) in the petrochemical industry but none have focused on the combined process of dehydrogenation and metathesis of mixed C₄/C₅ streams to produce mainly propylene and 1-hexene.

Although the emphasis of the present technological advancement is on the overall process and does not aim to claim any specific catalyst families or technologies to be used, it is noted that there is a broad range of catalyst technologies known to those of ordinary skilled in the art for the dehydrogenation and metathesis processes. There are several commercial (oxidative and non-oxidative) dehydrogenation processes developed for C₄ paraffins (IFP, Lummus, UOP), which can be applied to C₅₊ paraffins as well. Common catalysts used in these processes are based on supported Pt, in combination with promoters (e.g. Sn, Ga, K, etc.) or based on supported V and Cr-oxides. A wide variety of heterogeneous catalysts, including W, Re or Mo oxides supported on basic oxides, such as MgO or CaO, or other conventional catalyst supports such as SiO₂, Al₂O₃, ZrO₂, TiO₂, CeO₂ etc. can be used for the metathesis reaction described here. In addition, the use of homogeneous (e.g. including but not limited to Grubbs or Schrock type) catalysts for metathesis is also in scope for this invention. In general, the present technological advancement is useable with any dehydrogenation or metathesis catalyst system.

The metathesis reaction conditions are conventional, and can include a temperature of from about 50°C to about 600°C, preferably from about 200°C to about 400°C, a weight hourly space velocity (WHSV) of from about 3 to about 200, preferably from about 6 to about 40, and a pressure of from about 10 psig to about 600 psig, preferably from about' 30 psig to about 100 psig. The reaction may be carried out by contacting the olefin(s) with the catalyst in the liquid phase, gas phase or mixed phase depending on structure and molecular weight of the olefin(s) and specific reactor conditions.

It is noted that branched olefins are less reactive (or unreactive) in the metathesis reaction and are expected to pass through the process unreacted. If desired, they can be removed from the process by etherification with methanol with potential use in fuel applications (e.g. as MTBE or TAME fuel additives) and / or converted back into a high purity branched olefin stream by subsequent cracking.

For the evaluation of the overall process, the expected stream compositions were modeled based on the parameters below. The C₄/C₅ olefin feed composition was based on typical NGL composition of 2:1 butanes to pentanes and equilibrium dehydrogenation conversion was assumed at 550°C and 0.1 barg (yielding 50% conversion for butane and 60% conversion for pentanes and ~50 - 60% olefinicity of the stream after dehydrogenation). It is known that the metathesis reaction is a second order reaction with a statistical selectivity profile (e.g. the carbon number distribution primarily depends on the molar concentration of the reacting olefins). Therefore, to evaluate the expected metathesis product slate a simple statistical metathesis model was developed. To evaluate feasibility of the present technological advancement from product purity, distillation and recycle perspective, a simulation was built using process simulation software (PRO/II 10.1 SimSci).

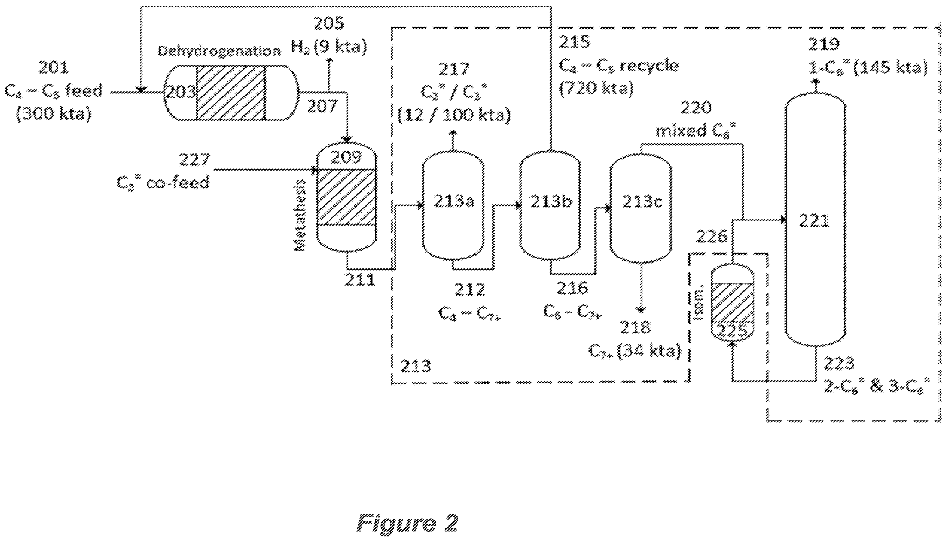

The statistical metathesis model assumed that a i-Cⱼ olefin will react with a n-Cₘ olefin to form a olefin of carbon number j + m - i - n and a second olefin of carbon number i + n. For example, 1-butene (1-C₄) will react with 2-pentene (2-C₅) to form a hexene and a propylene molecule. In this way, the (molar) concentration of the isomers in the feed were used to calculate the expected metathesis product distribution. C₄-C₅ paraffins and olefins were recycled through dehydrogenation and metathesis in the overall process simulation. The formed olefin isomers for C₄₊ were assumed to be at thermodynamic equilibrium after the respective high temperature dehydrogenation and metathesis reactors (assuming dehydrogenation conditions of 550°C and 0.1 barg and metathesis conditions of 300°C and 0.1 barg). The distillation of the resulting metathesis reactor product stream was simulated using the configuration as shown in **Figure 2** and the process simulation software mentioned above. An example of the conditions and stream compositions from the simulation, assuming no co-feeding of ethylene, is shown in

**Table .** For all distillation equipment, 30 trays and a reflux ratio of 3 was assumed and a specification of max. 0.1 wt.% of heavy products in the distillation overhead streams was set. It is noted that this is an example and that the distillation parameters can be further optimized to accommodate product purity and process and capital efficiency (e.g. heat integration and equipment sizing).

**Table 1**

| **Products (kta)** | **No C₂= co-feed (base case)** | **Metathesis C₂= recycle (0.8% C₂=)** | **5% C₂= cofeed** | **10% C₂= cofeed** | **20% C₂= cofeed** |
|---|---|---|---|---|---|
| H₂ | 8.8 | 8.8 | 7.8 | 7.1 | 6.6 |
| C₂= | 8.0 | | | | |
| C₃= | 105.0 | 120.8 | 172.3 | 208.5 | 249.0 |
| C₆= | 152.2 | 145.4 | 102.4 | 72.2 | 37.2 |
| C₇₊ | 26.0 | 25.0 | 17.5 | 12.2 | 7.2 |
| Total | 300.0 | 300.0 | 300.0 | 300.0 | 300.0 |

From the model it was identified that co-feeding ethylene will significantly shift the product distribution towards propylene and other lighter olefin products, which will allow tuning the amount of hexene vs. propylene that is formed, which and can be exploited in process operation. The base case here, assuming no co-feeding of ethylene, will maximize hexene yields but increase the size of the C₄ / C₅ recycle and C₇₊ stream.

Ethylene and propylene produced in the process is separated and purified on existing (or dedicated) separation trains. The separation(s) can be implemented by any method known to those skilled in the art. This method can be selected from distillation, extraction, absorption, membrane separation, precipitation or crystallization. Preferably, this separation is carried out by one or more fractionation columns.

Unreacted C₄/C₅ olefins and any remaining paraffins from the metathesis reactor are separated from the C₆ and C₇₊ olefins and recycled back to the dehydrogenation reactor.

The (linear) C₇₊ olefins produced can be further processed for use in chemical applications or as fuels. High purity 1-Hexene is collected from the overheads of a distillation column and remaining 2-hexene and 3-hexene are recycled back to an isomerization reactor.

Using the basic dehydrogenation and metathesis assumptions described above, it was calculated that a 300 kta sized feed stream, in the absence of co-fed ethylene, would yield 105 kta of propylene and 152 kta of 1-hexene, with the balance made up by hydrogen, ethylene and C₇₊ products.

**Table 2**

| | | | | | | | | |
|---|---|---|---|---|---|---|---|---|
| Stream Number | | 211 | 217 | 212 | 215 | 216 | 220 | 218 |
| Temperature | C | 50.0 | 17.0 | 101.9 | 27.2 | 93.9 | 90.4 | 120.9 |
| Pressure | BARG | 10.0 | 10.0 | 10.0 | 1.0 | 1.0 | 1.0 | 1.0 |
| | | | | | | | | |
| Total Mass Rate | T/h | 110.0 | 11.7 | 98.3 | 79.4 | 18.9 | 15.3 | 3.6 |
| | | | | | | | | |

| Component wt.% | | | | | | | | |
|---|---|---|---|---|---|---|---|---|
| Ethylene | | 1.1 | 10.5 | 0.0 | 0.0 | 0.0 | 0.0 | 0.0 |
| Propylene | | 9.6 | 89.4 | 0.1 | 0.1 | 0.0 | 0.0 | 0.0 |
| 1-Butene | | 7.6 | 0.1 | 8.5 | 10.5 | 0.0 | 0.0 | 0.0 |
| Butanes | | 19.2 | 0.0 | 21.5 | 26.6 | 0.0 | 0.0 | 0.0 |
| Trans-2-Butene | | 10.0 | 0.0 | 11.2 | 13.9 | 0.0 | 0.0 | 0.0 |
| Cis-2-Butene | | 7.7 | 0.0 | 8.7 | 10.7 | 0.0 | 0.0 | 0.0 |
| 1-Pentene | | 3.3 | 0.0 | 3.7 | 4.6 | 0.0 | 0.0 | 0.0 |
| Pentanes | | 6.4 | 0.0 | 7.2 | 8.9 | 0.0 | 0.0 | 0.0 |
| Trans-2-Pentene | | 12.0 | 0.0 | 13.4 | 16.6 | 0.0 | 0.0 | 0.0 |
| Cis-2-Pentene | | 5.8 | 0.0 | 6.5 | 8.0 | 0.0 | 0.0 | 0.0 |
| Hexenes | | 13.9 | 0.0 | 15.6 | 0.1 | 80.7 | 99.9 | 0.0 |
| C₇₊ | | 3.3 | 0.0 | 3.7 | 0.0 | 19.3 | 0.1 | 100.0 |

Figure 2 illustrates an exemplary embodiment using a combination of dehydrogenation and metathesis to produce C₃⁼ and 1-C₆⁼. A dehydrogenation unit **203** is configured to at least partially dehydrogenate a mixed C₄ and C₅ alkane stream **201** to generate at least a partial alkene stream **207.** The mixed C₄ and C₅ alkane stream **201** can be a natural gas liquid stream, light ends or complete of the fluid catalytic cracking light cracked naphtha stream or other fluid catalytic cracking product stream, a steam cracker C₄ or C₅ raffinate stream or a mixed C₄/C₅ stream from a biomass source. A natural gas liquid stream can be a NGL derived stream (e.g., a stream that is pretreated by distillation and/or selective hydrogenation/hydrofining to remove feed poisons or isomers. The mixed C₄ and C₅ alkane stream **201** can include linear and branched alkanes. While the mixed C₄ and C₅ alkane stream **201** is predominantly such alkanes, it can include other molecules, such as some alkenes. The mixed C₄ and C₅ alkane stream can include derivative streams from FCC and steam cracking and biomass (e.g., pretreated by distillation and/or selective hydrogenation/hydrofining).

Hydrogen stream **205** can be separated from stream **207** via conventional separation technology, which would be understood by those of ordinary skill in the art. Depending on the specific dehydrogenation technology applied, stream **207** can be optionally pretreated before metathesis to remove (e.g. by selective hydrogenation) any C₄ or C₅ diolefins from the stream selectively. A metathesis unit **209** is configured to apply metathesis to the alkene stream **207** to generate an effluent **211,** wherein the metathesis is optionally conducted in a presence of ethylene **227.** The metathesis can be conducted in the presence of ethylene, wherein the amount of ethylene supplied is controlled in order to vary amounts generated of the propylene and 1-hexene. Ethylene can be added to the process from a dedicated feed source (such as steam cracker ethylene or ethylene from ethanol dehydrogenation), or alternatively, any ethylene produced in the metathesis reaction from the metathesis of C₄/C₅ feed can be recycled back (e.g. from the cross metathesis of 1-C₄ and 1-C₅ olefins (and/or propylene)) to the metathesis reactor (for overall net zero ethylene make).

Using the basic dehydrogenation and metathesis assumptions described above, several ethylene co-feeding scenarios were calculated based on a 300 kta sized feed (C₄/C₅ and C₂⁼) stream.

Effluent **211** is fed into a separation unit **213** that is configured to separate the effluent from the metathesis into a C₄ and C₅ alkane fraction **215,** which is optionally recycled back to the dehydrogenating, propylene **217** (or a C₂⁼ / C₃⁼ mixed stream that is subsequently separated using conventional separation process, not shown), and 1-hexene **219.** Separation unit **213** can include a train of a plurality of distillation columns **213a, 213b, 213c** (with effluents **212** and **216** as depicted), and **221.** The number of distillation towers depicted in Fig. 2 is merely an example, and other configurations can be used along with the present technological advancement. The plurality of distillation columns can be configured to separate the effluent into a C₄-C₇₊ fraction, a C₆-C₇₊ fraction, a C₇₊ fraction, and a mixed C₆₌ fraction. The separation unit **213** can include a distillation column **221** that is configured to receive the mixed C₆₌ fraction, wherein the 1-hexene **219** is the overhead stream of the distillation column **221** and 2-hexene and 3-hexene **223** are recycled back to an isomerization reactor **225** and re-isomerized to an equilibrium mixture of 1-hexene, 2-hexene and 3-hexene **226,** and recombined with stream **220** for distillation in column **221,** as known and understood by those of ordinary skill in the art.

The term "comprising" is considered synonymous with the term "including." Likewise whenever a composition, an element or a group of elements is preceded with the transitional phrase "comprising", it is understood that we also contemplate the same composition or group of elements with transitional phrases "consisting essentially of," "consisting of", "selected from the group of consisting of," or "is" preceding the recitation of the composition, element, or elements and vice versa.

## Claims

1. A method, comprising:
at least partially dehydrogenating a mixed alkane stream, including at least C₄ and C₅ alkanes, to generate at least a partial alkene stream and hydrogen;
applying a metathesis reaction to the at least partial alkene stream to generate an effluent, wherein the metathesis reaction is optionally conducted in a presence of ethylene; and
separating the effluent from the metathesis reaction into propylene and 1-hexene.

2. The method of claim 1, wherein the metathesis is conducted in the presence of ethylene, and the method further comprises controlling an amount of the ethylene supplied for the metathesis reaction in order to vary amounts generated of the propylene and 1-hexene.

3. The method of claim 1, wherein the metathesis is conducted in the presence of ethylene recycled from a metathesis reactor product stream.

4. The method of claim 1, wherein the metathesis reaction is conducted without co-feeding the ethylene.

5. The method of claim 1, wherein the mixed C₄ and C₅ alkane stream is a natural gas liquid stream.

6. The method of claim 1, wherein the mixed C₄ and C₅ alkane stream is the light ends or complete of the fluid catalytic cracking light cracked naphtha stream or other fluid catalytic cracking product stream, a steam cracker C₄ or C₅ raffinate stream or a mixed C₄/C₅ stream from a biomass source.

7. The method of claim 1, wherein the separating includes separating the effluent into a C₄-C₇₊ fraction, a C₆-C₇₊ fraction, a C₇₊ fraction, and a mixed C₆₌ fraction.

8. The method of claim 6, wherein the method further comprises feeding the C₆₌ fraction to a distillation column, wherein the 1-hexene is overheads of the distillation column and 2-hexene and 3-hexene are recycled back to an isomerization reactor.

9. The method of claim 1, wherein the mixed C₄ and C₅ alkane stream includes linear and branched alkanes.

10. The method of claim 1, wherein the separating includes generating a C₄ and C₅ alkane fraction, which is optionally recycled back to the dehydrogenating.

11. A system, comprising:
a dehydrogenation unit configured to at least partially dehydrogenate a mixed alkane stream, including at least C₄ and C₅ alkanes, to generate at least a partial alkene stream and hydrogen;
a metathesis unit configured to apply a metathesis reaction to the alkene stream to generate an effluent, wherein the metathesis reaction is optionally conducted in a presence of ethylene; and
a separation unit configured to separate the effluent from the metathesis reaction into propylene and 1-hexene.

12. The system of claim 11, wherein the separation unit includes a train of a plurality of distililation cloumns.

13. The system of claim 12, wherein the plurality of distillation columns are configured to separate the effluent into a C₄-C₇₊ fraction, a C₆-C₇₊ fraction, a C₇₊ fraction, and a mixed C₆₌ fraction.

14. The system of claim 13, wherein the separation unit includes a distillation column configured to receive the mixed C₆₌ fraction, wherein the 1-hexene is overheads of the distillation column and 2-hexene and 3-hexene are recycled back to an isomerization reactor.

15. The system of claim 13, wherein the separation unit generates a C₄ and C₅ alkane fraction, which is optionally recycled back to the dehydrogenation unit.

## Patentansprüche

1. Verfahren, bei dem:
ein gemischter Alkanstrom, der mindestens C₄- und C₅-Alkane enthält, zumindest teilweise dehydriert wird, um mindestens einen Teil-Alkenstrom und Wasserstoff zu erzeugen;
der mindestens eine Teil-Alkenstrom einer Metathesereaktion unterworfen wird, um ein Reaktionsprodukt zu erzeugen, wobei die Metathesereaktion gegebenenfalls in Gegenwart von Ethylen durchgeführt wird; und
der Produktstrom aus der Metathesereaktion in Propylen und 1-Hexen getrennt wird.

2. Verfahren nach Anspruch 1, wobei die Metathese in Gegenwart von Ethylen durchgeführt wird und bei dem Verfahren weiterhin eine Menge des für die Metathesereaktion zugeführten Ethylens kontrolliert wird, um die erzeugten Mengen an Propylen und 1-Hexen zu variieren.

3. Verfahren nach Anspruch 1, wobei die Metathesereaktion in Gegenwart von Ethylen durchgeführt wird, das aus einem Produktstrom eines Metathesereaktors zurückgeführt wird.

4. Verfahren nach Anspruch 1, wobei die Metathesereaktion ohne Mitzuführung von Ethylen durchgeführt wird.

5. Verfahren nach Anspruch 1, wobei der gemischte C₄- und C₅-Alkan-Strom ein flüssiger Erdgasstrom ist.

6. Verfahren nach Anspruch 1, wobei der gemischte C₄- und C₅-Alkanstrom die leichten Fraktionen oder der gesamte Strom des durch katalytisches Fluidcracken gewonnenen leichten Naphthastroms oder eines anderen Produktstroms aus dem katalytischen Fluidcracken, ein C₄- oder C₅-Raffinatstrom aus einem Dampfcracker oder ein gemischter C₄-/C₅-Strom aus einer Biomassequelle ist.

7. Verfahren nach Anspruch 1, wobei das Trennen das Aufteilen des Abstroms in eine C₄- bis C₇₊-Fraktion, eine C₆- bis C₇₊-Fraktion, eine C₇₊-Fraktion und eine gemischte C₆₌-Fraktion umfasst.

8. Verfahren nach Anspruch 6, bei dem weiterhin die C₆₌-Fraktion einer Destillationskolonne zugeführt wird, wobei das 1-Hexen als Kopfprodukt der Destillationskolonne anfällt und 2-Hexen und 3-Hexen in einen Isomerisierungsreaktor zurückgeführt werden.

9. Verfahren nach Anspruch 1, wobei der gemischte C₄- und C₅-Alkanstrom lineare und verzweigte Alkane enthält.

10. Verfahren nach Anspruch 1, wobei die Trennung die Erzeugung einer C₄- und C₅-Alkanfraktion umfasst, die optional in die Dehydrierungsstufe zurückgeführt wird.

11. System, umfassend:
eine Dehydrierungsanlage, die konfiguriert ist, um einen gemischten Alkan-Strom, der mindestens C₄- und C₅-Alkane enthält, zumindest teilweise zu dehydrieren, um mindestens einen Teil-Alkenstrom und Wasserstoff zu erzeugen;
eine Metatheseeinheit, die konfiguriert ist, um den Alkenstrom einer Metathesereaktion zu unterwerfen, um einen Abstrom zu erzeugen, wobei die Metathesereaktion gegebenenfalls in Gegenwart von Ethylen durchgeführt wird; und
eine Trenneinheit, die konfiguriert ist, um das Produkt der Metathesereaktion in
Propylen und 1-Hexen zu trennen.

12. System nach Anspruch 11, wobei die Trenneinheit eine Reihe mehrerer Destillationskolonnen umfasst.

13. System nach Anspruch 12, wobei die mehreren Destillationskolonnen konfiguriert sind, um den Abstrom in eine C₄ bis C₇₊ -Fraktion, eine C₆ bis C₇₊ -Fraktion, eine C₇₊ -Fraktion und eine gemischte C₆₌-Fraktion zu trennen.

14. System nach Anspruch 13, wobei die Trenneinheit eine Destillationskolonne umfasst, die konfiguriert ist, um die gemischte C₆₌ -Fraktion aufzunehmen, wobei das 1-Hexen als Kopfprodukt der Destillationskolonne anfällt und 2-Hexen und 3-Hexen in einen Isomerisierungsreaktor zurückgeführt werden.

15. System nach Anspruch 13, wobei die Trenneinheit eine C₄- und C₅-Alkanfraktion erzeugt, die optional in die Dehydrierungsanlage zurückgeführt wird.

## Revendications

1. Procédé, comprenant :
déshydrogéner au moins partiellement un flux d'alcanes mixtes comportant au moins des alcanes en C₄ et en C₅ pour générer au moins un flux d'alcène partiel et de l'hydrogène ;
appliquer une réaction de métathèse au flux d'alcène au moins partiel pour générer un effluent, la réaction de métathèse étant éventuellement conduite en une présence d'éthylène ; et
séparer l'effluent de la réaction de métathèse en propylène et 1-hexène.

2. Procédé selon la revendication 1, dans lequel la métathèse est conduite en présence d'éthylène, et le procédé comprend en outre la régulation d'une quantité d'éthylène fournie pour la réaction de métathèse afin de faire varier des quantités générées du propylène et du 1-hexène.

3. Procédé selon la revendication 1, dans lequel la métathèse est conduite en présence d'éthylène recyclé à partir d'un flux de produit de réacteur de métathèse.

4. Procédé selon la revendication 1, dans lequel la réaction de métathèse est conduite sans co-alimentation de l'éthylène.

5. Procédé selon la revendication 1, dans lequel le flux d'alcanes mixtes en C₄ et en C₅ est un flux liquide de gaz naturel.

6. Procédé selon la revendication 1, dans lequel le flux d'alcanes mixtes en C₄ et en C₅ est les fractions légères ou complètes du flux de naphta de craquage léger catalytique de fluide ou d'un autre flux de produit de craquage catalytique de fluide, d'un flux de raffinat en C₄ ou en C₅ de vapocraqueur ou d'un flux mixte en C₄/C₅ provenant d'une source de biomasse.

7. Procédé selon la revendication 1, dans lequel la séparation comprend la séparation de l'effluent en une fraction en C₄-C₇₊, une fraction en C₆-C₇₊, une fraction en C₇₊, et une fraction mixte en C₆₌.

8. Procédé selon la revendication 6, dans lequel le procédé comprend en outre l'alimentation de la fraction en C₆₌ à une colonne de distillation, dans lequel le 1-hexène est une tête de colonne de distillation et le 2-hexène et le 3-hexène sont recyclés à nouveau dans un réacteur d'isomérisation.

9. Procédé selon la revendication 1, dans lequel le flux d'alcanes mixtes en C₄ et en C₅ comprend des alcanes linéaires et ramifiés.

10. Procédé selon la revendication 1, dans lequel la séparation comprend la génération d'une fraction d'alcanes en C₄ et en C₅, qui est éventuellement recyclée à nouveau vers la déshydrogénation.

11. Système, comprenant :
une unité de déshydrogénation configurée pour déshydrogéner au moins partiellement un flux d'alcanes mixtes, comprenant au moins des alcanes en C₄ et en C₅, pour générer au moins un flux d'alcène partiel et de l'hydrogène ;
une unité de métathèse configurée pour appliquer une réaction de métathèse au flux d'alcène afin de générer un effluent, la réaction de métathèse étant éventuellement conduite en une présence d'éthylène ; et
une unité de séparation configurée pour séparer l'effluent de la réaction de métathèse en propylène et en 1-hexène.

12. Système selon la revendication 11, dans lequel l'unité de séparation comprend un train d'une pluralité de colonnes de distillation.

13. Système selon la revendication 12, dans lequel la pluralité de colonnes de distillation est configurée pour séparer l'effluent en une fraction en C₄-C₇₊, une fraction en C₆-C₇₊, une fraction en C₇₊, et une fraction mixte en C₆₌.

14. Système selon la revendication 13, dans lequel l'unité de séparation comprend une colonne de distillation configurée pour recevoir la fraction mixte en C₆₌, dans lequel le 1-hexène est une tête de colonne de distillation et le 2-hexène et le 3-hexène sont recyclés à nouveau dans un réacteur d'isomérisation.

15. Système selon la revendication 13, dans lequel l'unité de séparation génère une fraction d'alcanes en C₄ et en C₅, qui est éventuellement recyclée à nouveau vers l'unité de déshydrogénation.
